# EUROPEAN PATENT APPLICATION

(11) **EP 4 039 338 A1**
(43) Date of publication of application: **10.08.2022**
(21) Application number: 19946532.9
(22) Date of filing: 17.10.2019
(51) Int. Cl.: A61Q 11/00, A61K 6/60, A61K 6/65, A61K 6/884

(54) **ORAL-HEALTH COMPOSITION AND PREPARATION METHOD FOR ORAL-HEALTH COMPOSITION**

(30) Priority: 27.09.2019 BR 102019020257
(71) Applicant: MMF&T Desenvolvimento Tecnológico e Inovação Ltda., 12247-902 São José dos Campos - SP (BR); Vilhena, Fabiano Vieira, 17018-520 Bauru - SP (BR)
(72) Inventor: VILHENA, Fabiano Vieira, 17018-520 Bauru - SP (BR)
(74) Representative: Sarpi, Maurizio
(86) International application number: PCT/BR2019/050449
(87) International publication number: WO 2021/056086

(57) **Abstract**

This patent application refers to oral health composition and preparation process thereof, suitable for direct administration into the oral cavity by specialists or otherwise, wherein the formulations have a wide action spectrum due to self-activation and continuous formation of reactive oxygen, with no need for external activation by light, chemicals or electricity, except for air oxygen, said oral health compositions acting through the synergistic action of air oxygen with octa-carboxy or tetra-carboxy (metal-phthalocyanines) being functional dyes capable of promoting self-activation and continuous production of reactive oxygen in the presence of molecular oxygen in suitable, safe and effective amounts over odor-making substances, microorganisms and over coagulation/healing mechanisms, among other properties of interest for maintenance of oral health, which may be used directly by the patient or by a professional.

## Description

### FIELD OF APPLICATION

This patent application relates to the fields of human needs, medical sciences, dental sciences and hygiene; more specifically, it relates to oral compositions with continuous and localized formation of reactive oxygen, granting antimicrobial and cleaning (increased teeth and toothbrush cleanliness, and subsequent increased brightness, whiteness, and anti-tartar properties) activities to formulations/compositions such as tissue regeneration, anti-malodor, anti-ulcerative, remineralization (favors remineralization due to anti-bacterial properties) among others.

### INTRODUCTION

This invention patent application is directed to innovative products aimed at oral health and suitable for direct administering to the oral cavity by specialists and non-specialists, wherein the formulations have a broad action spectrum due to self-activation and continuous and localized formation of reactive oxygen, with no need of external activation by light, chemicals or electricity, except for air oxygen. Such oral health compositions, through synergistic action of air oxygen with octa-carboxy or tetra-carboxy (metal-phthalocyanines), characterized as functional dyes, are capable of promoting self-activation and continuous production of oxygen that is reactive in the presence of molecular oxygen in suitable, safe and effective amounts on odor-generating substances, microorganisms, on coagulant/healing mechanisms, among other properties of interest for maintenance of oral health, which may be used directly by the patient or by a professional.

This application includes toothpaste compositions in all physical forms of administration and presentation, oral antiseptics on all its physical forms of administration and presentation, gels, pastes, mouthwashes, varnishes or even a combination of two or more compositions. Some of the actions rely in control of oral biofilm, which consequently results in prevention and decrease of tooth and periodontal decay; tartar control, prevention and/or decrease of odors and halitosis; increase in tissue healing; control of oral ulcerations; increased cleanliness, brightness and decrease of tooth darkening and preservation of toothbrush cleaning.

### STATE OF THE ART

Currently, there are several innovations available for maintenance of health, which consequently have increased self-esteem and life expectancy of people. In the field of oral health, in the area of application of said patent application, this "increase of oral tissue protection" is related to technologies for control of oral cavity diseases, particularly in controlling biofilm, prevention and decrease of tooth and periodontal decay, tartar control, increase of tissue healing speed, prevention and/or decrease of foul odor, in addition to increase in cleanliness, brightness and decrease of darkening of biological oral structures, such as gums and teeth, but also functional auxiliary structures such as orthodontic appliances, partial and full prosthetics, in addition to the toothbrush itself.

All this is accessible via self-care by the individual, or yet by technologies available to area professionals.

Mouth cleanliness is granted by cleaning compositions such as toothpaste and mouthwashes which, when helped by dental floss and toothbrushes, clean and highlight the smile. Incorporated to these compositions, ingredients such as cosmetic detergents, abrasive silicas, anti-tartar and whitening agents, active agents, among others, promote mouth cleanliness and, therefore, increased teeth brightness and whiteness (Lippert 2013). Still on the subject, it may also be said that there are products available on the market for cleaning the tongue, denture and even cleaning the toothbrush itself.

The increase in individual self-esteem may also be related to ingredients that prevent foul odor, in addition to ingredients that grant flavor and scents to oral compositions.

Anti-malodor agents are typically based on the chemical reaction with volatile sulfur compounds (VSCs) such as methyl mercaptan and hydrogen sulfide. Zinc salts are more commonly used, since zinc not only has antimicrobial properties, but it is also capable of reacting with VSCs, thus transforming them into non-volatile zinc salts (zinc sulfide is one of the least soluble compounds) (Lippert 2013).

The consumer market features a product developed for tongue cleaning and hygiene, with the Zinc Chloride as the active ingredient for neutralization of halitosis (when originated by tongue plaque). Tongue plaque (white plaque that covers the surface of the tongue) is made of food scraps, shed mucosa cells and bacteria. It is a common cause of halitosis, since it provides release of sulfur compounds in the oral cavity.

One may also acquire several types of mouth sprays with aromas for control of halitosis.

Still regarding available technologies, many are not new, but effectively contribute to oral health, among which are those containing active ingredients such as chlorhexidine, triclosan, cetylpyridinium chloride, gantrez, thymol, menthol, eucalyptol. These examples have an established literature regarding antimicrobial activities and are largely used in oral compositions. This way, gels, solutions, varnishes including Chlorhexidine are already available in the market for domestic and/or professional use and, in a way, have greatly contributed to the health of populations (antimicrobial and healing effect).

More recently, a technology with Ethylenediaminetetraacetic Acid (EDTA) as an active ingredient was released in a dental gel for microbial control in the oral cavity. According to the manufacturer, the dental gel was capable of removing 2x more microorganisms than the market leading brand of toothpaste.

According to Cabezas & Fernández, 2018, it is still possible to attest that some antimicrobial agents (Triclosan, for example), as being "biofilm modifiers", may also be considered "dental remineralizers".

However, not all antimicrobial agents are 100,000% acceptable.

In spite of its wide and diversified use, Triclosan is one example. In September 2016, the Food and Drug Administration (FDA), the North American regulatory agency equivalent to Anvisa in Brazil, announced that all cosmetic antiseptic products containing Triclosan must be taken out of market until September 2017. This action was taken based on the inefficiency and unproven safety for human use.

Chlorhexidine follows the same path due to its residual effect, in addition to the undesired effect of tooth darkening with prolonged use.

"Another antimicrobial therapy is Antimicrobial Photodynamic Therapy - PDT. PDT is a therapy that uses a light source and a photosensitizer (PTS) in photo-inactivation of microorganisms. PDT is not considered a replacement to antimicrobial drugs or any conventional treatment, but an important complementary treatment modality for localized mouth infections, mainly for cases of resistant microorganisms. The sensitivity of bacteria, viruses and fungi to PDT suggests its applicability to shallow localized infections and known microbiota. Low cost, minimum side effects and reduced recurrence probability are the main advantages of the therapy, in addition to technical simplicity and lack of microbial resistance risk" (summary of article from Paula Eduardo 2015 et *al*.).

"The PDT photosensitization mechanism consists in interaction of light with the photosensitizer and oxygen, generating free radicals that cause severe damage to the microbial cells, leading to their death. The light is responsible for exciting the photosensitizing (PTS) agent that interacts with nearby molecules through two mechanisms. The photosensitizing in its excited state may act by removing a hydrogen atom from a molecule of the biological substrate (phospholipids, cholesterol, proteins, among others) or transferring electrons, generating radical ions that tend to react with oxygen in its fundamental state. For PDT to be effective, it is invaluable that the light source interacts with PTS. Literature features several PTS acting effectively in PDT, one of them being the phenotiazinic dyes such as Methylene Blue (MB) which are commonly used in Dentistry" (excerpt from article from Paula Eduardo 2015 et *al*.)*.*

Over the last decades, compounds considered as the second generation of dyes were developed, with diagnostic and therapeutic purposes, among which one of the most promising alternatives to PDT is included, the group of phthalocyanines.

From its synthesis and accidental characterization in Scotland, in the 1920s, phthalocyanines have been under intense research as dyes and pigments.

Phthalocyanines are synthetic dyes similar to porphyrins and structurally belong to the azaporphyrin class. PDTs are classified as second generation PTS, whose lipophilicity would contribute to its location in plasmatic membranes, microsomes and mitochondria, and, consequently, for photodynamic action.

"Zinc phthalocyanines (ZnPc) are among the most promising sensitizers of this group" (text fully taken from dissertation *Avaliação dos Efeitos da Terapia Fotodinâmica Antimicrobiana com* o *Fotossensibilizador Ftalocianina-Glucamina como Terapia Adjunta no Tratamento da Doença Periodontal Induzida em Ratos* [Evaluation of the Effects of Antimicrobial Photodynamic Therapy with Phthalocyanine-Glucamine Photosensitizer as an Associate Therapy in Treatment of Induced Periodontal Disease in Rats] - Corbi 2014) .

Phthalocyanines feature very low solubility on most of organic solvents commonly used as a result of strong π-π interactions of the aromatic macrocyclic rings of phthalocyanines in crystalline state. On one hand, the low solubility is a desired quality in compounds used as pigments, but, on the other hand, it brings several limitations to many other potential applications and prevents solution testing. Therefore, replaced phthalocyanines are studied mainly in solution, as its properties depend on the type of substituting groups, their number and arrangement at the Pc ligand. NACKIEWICZ 2014, Octacarboxy phthalocyanines - compounds of interesting spectral, photochemical and catalytic properties.

Longo and coworkers (2012) have developed a clinical protocol involving the application of PDT mediated by aluminum-phthalocyanine chloride (AlClPc) encapsulated in cationic liposomes against cariogenic bacteria in dental carious lesions. Cationic liposomes were used to spread AlClPc preferably on bacterial cells due to the high density of negative charges on the surfaces of this cell type. The main results have shown that the cationic liposome loaded with AlClPc was preferably incorporated by bacterial cells in comparison to eukaryotic dental pulp cells, and was effective in reducing the microbial load of bacterial cultures. In addition, the clinical study has shown an average reduction of 82,000% of the total of bacteria in cavities treated after application of PDT. For authors, results featured in this study have shown that the PDT antimicrobial protocol mediated by cationic liposomes including AlClPc is safe for clinical application and effective in reducing bacterial load in carious lesions. (Longo 2012 - Photodynamic therapy disinfection of carious tissue mediated by aluminum-chloride-phthalocyanine entrapped in cationic liposomes: an *in vitro* and clinical study).

Corbi, 2014, has concluded that PDT with phthalocyanine-glucamine PTS was effective in the treatment of periodontal disease induced in rats. Thus, phthalocyanine-glucamine may be considered a promising photosensitizer.

Tapajós and coworkers (2008) have evaluated the performance of phthalocyanines as PTS in PDT on carcinogenic oral cells. Results have indicated a 95,000% reduction in cell viability for tumoral cells, in addition to morphological changes. As concluded, authors have reinforced the efficiency of activated phthalocyanines by PDT.

De Paula Eduardo 2015 et *al.* state that, in addition to antimicrobial activity, PDT has also been used in tissue regeneration (healing and treatment of ulcerative tissue). Regarding tissue regeneration, products that do not use phthalocyanines have yielded excellent results. For example, products with urea peroxide as the main active ingredient may be found. The action mechanism of oral compositions, although also being based in the release of reactive oxygen such as in oral compositions that are object of this patent application, this one is completely different, since when the active ingredient of the products is fully consumed, the action stops.

In contrast, oral compositions of this patent application include self-activated carboxy-metal-phthalocyanines (octa or tetra) in its formulation, which are constant makers of reactive oxygen when in the presence of molecular oxygen.

It is also possible to find the Triamcinolone Acetonide Cream, a synthetic corticosteroid which has antiinflammatory effect acting on the temporary relief of symptoms associated with oral inflammatory lesions and ulcerative lesions resulting from trauma.

### EVOLUTION OF THE STATE OF THE ART

Regarding the current state of products already in the market, this patent application features the same physical characteristics and main ingredients (such as thickener (Gel), dye, sweetener, essence, foam-forming surfactant, base and solvent (varnish), etc.

However, based on the state of the art, it is possible to attest that, currently, there are no oral compositions capable of joining all desired actions/effects (antimicrobial, anti-ulcerative, tissue and dental regeneration, anti-malodor and whitening) in unison, associated to functional self-activated carboxy-metal-phthalocyanines (octa or tetra). Moreover, when the action/effect takes place individually, action mechanisms are entirely different when compared to the aforementioned patent object, as previously demonstrated.

In this context, it should be mentioned that scientific articles and patents focused on phthalocyanines and derived in oral treatment or from oral health explore the optical whitening properties and as a dye, but mainly as photosensitizers for application via the photodynamic therapy technique (activity dependent on light), as demonstrated in the patents listed below.

The state of the art comprises some documents related to phthalocyanine, which are shown as follows.

Firstly, document WO 2012/123241 A2 - "ORAL CARE COMPOSITIONS" features a toothpaste formula for teeth whitening that particularly provides the use of "phthalocyanine blue pigment" as a whitening agent.

The use of phthalocyanine blue pigment in the patent application WO 2012/123241 A2 works only as an optical bleaching effect for teeth, with no additional action.

Whereas this patent application provides the use of functional self-activated carboxylated metal phthalocyanines (octa or tetra), Active Ingredient with effective *in situ* formation of reactive oxygen, which leads to deterioration of organic matter and removal of outer stains, consequently leading to better teeth cleanliness. As a result, teeth become brighter, with a natural-looking color, due to more effective cleaning.

The use of phthalocyanine, as previously described, is widely explored as a dye.

Another relevant document is CA2512159C - "ORAL CARE COMPOSITIONS AND METHODS" - provides the use of phthalocyanine as green dye (phthalocyanine green) and CN107028783A - "ORAL CARE PRODUCT AND MOUTH WASH CONTAINING CURCUMIN AND PREPARATION METHOD OF MOUTH WASH" - provides the use of phthalocyanine as blue dye (phthalocyanine blue pigment).

Another market example in oral health is the toothpaste, which uses "phthalo blue" - CI74160 as a dye.

US6204234B1 and US6734155B1 -"CLEANING COMPOSITIONS COMPRISING A SPECIFIC OXYGENASE" - anticipate the use of phthalocyanines as enzymatic photoactivated bleaching agents, differently from the invention application requested herein, with multiple actions activated autonomously, with no light presence.

Examples of patents that anticipate the action of phthalocyanine for malodor stripping in the oral cavity - US6197070B1 - "DETERGENT COMPOSITIONS COMPRISING ALPHA COMBINATION OF A-AMYLASES FOR MALODOR STRIPPING" and "ORAL CARE REGIMEN SAND DEVICES" approach the need for activation of its properties through light. In this invention patent application, the phthalocyanine used does not require an activator (self-activated).

Another document found is GB2343187A - "DI - & octa-sulpho-phthalocyanine & naphthalocyanine dye derivatives for use in tissue demarcation, imaging & diagnosis of tumor cells & diseased lymph nodes" - addresses the use of phthalocyanine as a dye composite for use with light (phthalo-photoactivated) in the PDT.

Although said patent provides, since 1998, the application of use in dental/gingival problems, this patent application differs mainly by the use of functional self-activated carboxylated metal phthalocyanines (octa or tetra), which does not require the use of common phthalocyanine by light.

Patent EP0484027A1 - "POLY SUBSTITUTED PHTHALOCYANINES" - anticipates the use of substituted phthalocyanines for generation of singlet oxygen in at least one of the peripheral carbon atoms of the core of the phthalocyanine structure. However, this patent provides the generation of singlet oxygen from the phthalocyanine compounds only under influence of an external light source (electromagnetic radiation) particularly in the range from 60 to 770 nm - (use under condition of PDT).

As previously demonstrated, the singular electronic and molecular structure of Phthalocyanines make them photochemically, electrochemically and catalytically active, considering that said properties may be controlled/adjusted in function of the center metallic ion and the state of oxidization, the substitutes in the outer ring and also in function of the mode and degree of intermolecular association, generating the various properties that are being explored in science and technology.

Thus, non-substituted metal phthalocyanines with iron, nickel, cobalt, zinc, magnesium ions, etc. are produced on a large scale to address demands, mainly from the dye industry, in view of the simplicity and increased efficiency of cyclization reactions. However, the efficiency of said fundamental reaction depends on the type and number of substitute groups present in the outer ring, becoming significantly lower in case of reactive substitutes such as carboxylic acids/carboxylates. Indeed, results tend to diminish even further due to the number of carboxylate groups bound to the ring, rendering these less economically competitive. Nonetheless, said molecules feature interesting catalytic properties, particularly regarding what concerns oxidization reactions, more specifically, molecular oxygen activation reactions. This way, interesting biocide and odor removal properties were reported for the development of new applications and products. This way, more efficient large-scale production processes of carboxylated metal phthalocyanines, and consequently with less production costs, may enable the development of more products and applications, particularly as dyes, pigments and functional additives for various sectors.

This is proposed by WO 2018/064735 A1 - "PRODUCTION PROCESS OF CARBOXY-METAL-PHTHALOCYANINES FOR USE IN PRODUCTION OF DYES AND PIGMENTS". WO 2018/064735 A1, property of company Golden Technology, anticipates the synthesis/production mode of the main active ingredient of this invention application. The oral compositions requested herein benefit from carboxy-metal-phthalocyanines, which feature a higher efficiency in production of said molecule with lower cost, even lower residue production levels and a high degree of purity when compared to conventional processes.

### AIM OF THE INVENTION

The invention refers to oral compositions characterized by a wide spectrum of action based in self-activation and continuous and localized formation of reactive oxygen, with no requirement of external activation by light, chemicals or electricity, except for molecular hydrogen. Such oral health compositions via synergistic action of air oxygen with the functional self-activated carboxy-metal-phthalocyanines (octa or tetra), continuously generates suitable and safe amounts of reactive oxygen, effective on odor generating substances, microorganisms, on coagulation/healing mechanisms, among other properties of interest for maintenance of oral health, which may be used directly by the patient or a professional. In a joint and simultaneous form, this invention patent application acts on control of oral biofilm, which consequently results in prevention and decrease of tooth and periodontal caries, tartar control, prevention and/or decrease of odors and halitosis, increase in tissue healing, control of oral ulcerations, increased cleanliness, brightness and decrease of tooth darkening and preservation of toothbrush cleaning.

On the antimicrobial potential of phthalocyanines applied to "oral care", it is understood that phthalocyanine is an aromatic macrocyclic composite, widely used as dyes, featuring an indirect antimicrobial action (Photodynamic Therapy - PDT), direct antimicrobial action (Antibacterial).

The "oral care", in direct comparison with Phthalocyanine, features a direct antimicrobial action (Photodynamic Therapy - PDT) towards *Porphyromonas gingivalis* and further provides indirect antimicrobial action (photodynamic therapy - PDT) on the biofilm of *Porphyromonas gingivalis*; indirect antimicrobial action (Cariogenic bacteria and periodontal pathogens), with phthalocyanine being applied in toothpaste (paste and dental foam) and dental floss.

Studies were carried out for the improvement of antimicrobial potential of phthalocyanines, in an intrinsic aim of developing toothpastes, such as dental creams and dental foams including phthalocyanine for in vitro studies and possibly also in vivo studies.

For this purpose, the following microorganisms were used: *Streptococcus mutans* (ATCC 25175); *Candida albicans* (ATCC 10231); *Enterococcus faecalis* (ATCC 29212); and *Staphylococcus aureus* (ATCC 6538).

As initial results, it was revealed that phthalocyanine featured a microbicidal action for all microorganisms present in different concentrations and timespans studied, with full inhibition of microbial growth after direct contact with phthalocyanine.

The following step verified the cytotoxicity of phthalocyanine in face of human cell lineages.

Afterwards, toothpastes and its whites (negative control) were developed for the study sequence, attesting the anti-biofilm potential of phthalocyanine and toothpastes against the studied microorganisms and also studied the microbial action of phthalocyanine against negative Gram bacteria (*Escherichia coli*, *Pseudomonas aeruginosa and Klebsiella pneumoniae).*

In order to confirm the aforementioned statements, laboratory tests and clinical cases that demonstrate the effects were developed.

Through laboratory tests the positive antimicrobial effect for the main microorganisms that cause dental caries was measured, in addition to fungi and opportunist bacteria.

Below are some clinical cases (data not disclosed).

In the first case, a female patient above 50 years of age that used orthodontic appliances for a period of 1 year. After the use of one of the formulations, object of this patent application, straight on the first day of use she reported increased cleanliness, decreased gum and lip inflammation due to the brackets, teeth whitening, decrease of halitosis. She used to use another composition to brush her teeth. When comparing the two compositions, a favorable action of the invention application was verified regarding faster healing, reduction of ulcers (normally many would appear when the orthodontic appliance was tightened and lasted around 10 days. in addition, with the application of the object proposed in this patent application, less events of stomatitis occurred, and less painful, taking around 5 days to disappear) After tightening the orthodontic appliance, usually 10 days were needed for the mouth to be back to its normal state, with use of normal toothpaste.

The second case refers to a male adult patient, above 70 years of age, with gum inflammation and bleeding, halitosis and accumulation of bacterial plaque. Indication of periodontal treatment. With continuous use of the object of this patent, gum bleeding disappeared in the first few days, and a much healthier gum was obtained. The halitosis disappeared.

The third case refers to a female adult patient, above 80 years of age, which experienced control of oral thrush (ulcers) in 2 to 3 days after use of the oral compositions described herein.

The fourth case referred to male adult patients, smokers, around 45 years of age. They reported an increased cleanliness of the toothbrush, as well as full removal of the flavor and foul odor caused by cigarettes.

The fifth case referred to a male adult patient, around 45 years of age, which reported anti-tartar activity, increase brightness and "whiteness", as well as bleaching of the teeth.

The sixth case refers to a female adult patient, above 60 years of age, wearing an orthodontic appliance. She reported increased cleaning activity and bleaching, both of the appliance and the teeth, which was confirmed by the dental surgeon responsible for maintenance of the appliance. She also reported healthier gums, without inflammation.

The seventh case referred to a male adult patient, above 60 years of age. He reported anti-tartar activity, increased brightness and whitening/bleaching.

Lastly, the eighth case referred to a female adult patient, above 40 years of age, which works as a dental surgeon. She reported using the composition in some patients, the oral compositions for topical application and for home use by the patient. She reported a major improvement in oral health conditions on all patients, which, according to her, was easily perceivable.

In an unprecedented fashion, the invention patent application proposed herein promotes activities in the oral cavity listed below in a combined and simultaneous manner, as opposed to the state of the art, which strikes separately one or other function.

Antimicrobial - via formation and release of reactive oxygen, the object addressed in said invention patent application is capable of acting as an antimicrobial agent caused by the action of the oxygen generated by the functional self-activated carboxy-metal-phthalocyanines (octa or tetra).

Tissue and ulcer healing - in this case, the action of oral compositions address chemical cauterization, caused by release of reactive oxygen by action of functional self-activated carboxy-metal-phthalocyanines (octa or tetra), causing tissue oxidization, which leads to a repair.

Cleaning - via formation and release of reactive oxygen, the object addressed in said invention patent application is capable of acting as a cleaning agent, deactivating or solubilizing organic matter (bacteria and food scraps). In addition, the action mechanism of said oral compositions, object of this patent application, may also be associated to modification of biofilm due to the fixing of functional self-activated carboxy-metal-phthalocyanines (octa or tetra) on the oral connection locations (teeth and mucosa) which would be used for formation of biofilm. This happens because there is an electrostatic interaction between functional self-activated carboxy-metal-phthalocyanines (octa or tetra) negatively loaded and the enamel hydroxyapatite, a mineral that is part of the tooth enamel, positively loaded.

Foul odor - reaction and elimination of volatile compounds with reactive oxygen formed by action of the functional self-activated carboxy-metal-phthalocyanines (octa or tetra).

Anti-tartar - due to the action of the reactive oxygen formed through the functional self-activated carboxy-metal-phthalocyanines (octa or tetra), the oxidization of the tartar biofilm takes place, turning it even more soluble. In addition, it acts on the fixation of the initial bacterial plaque process, preventing the start of the formation of a dental stone.

Bleaching - coloring via cleaning and optical means. As for cleaning, it is related both to the formation and release of active oxygen, and to the binding of functional self-activated carboxy-metal-phthalocyanines (octa or tetra) directly to the tooth, changing the formation of the acquired film (which causes enamel stain), leaving the teeth as natural as possible; it might also be associated to the change of biofilm due to the fixing of functional self-activated carboxy-metal-phthalocyanines (octa or tetra) on oral connection locations (tooth and mucosa) which would be used for formation of biofilm. This happens because there is an electrostatic interaction between functional self-activated carboxy-metal-phthalocyanines (octa or tetra) negatively loaded and the enamel hydroxyapatite, a mineral that is part of the tooth enamel, positively loaded. This connection process to the dental structure may change the interaction of light with the tooth, making it brighter.

As previously stated, other phthalocyanines in the state of the art require external activation (photo or electroactivation), or are incapable of attaining so many simultaneous effects, as they were not designed for all these purposes.

Oral compositions described in this invention patent application with activities benefited by the formation and release of reactive oxygen, enabled by functional self-activated carboxy-metal-phthalocyanines (octa or tetra), which preferably use metals such as Fe and Co, or both in its formulation, or the association of other metals, may be administered in encapsulated form or not, molecular or not.

It is still possible to state that, when other active ingredients are added, the current compositions of this invention patent application, enhancing actions shall take place and shall enable more effects to the compositions, depending on indication. Below is a table with complementary effects:

| Additional components in compositions | Activity |
|---|---|
| Fluorine, Calcium and Phosphates complexes | Dental Remineralization |
| Fluorine, Calcium, Phosphate complexes and Potassium and Strontium compounds | Desensitizer |
| Pyrophosphated Compounds, abrasive silicas and other bleaching agents | Bleaching |

Regarding the current state of the products already in the market, said compositions have the same physical characteristics and main ingredients such as thickener, sweetener, essence, surfactants, base and solvent, etc., added of the main active ingredient, the functional self-activated carboxy-metal-phthalocyanines (octa or tetra).

The patent application described herein describes toothpaste oral compositions in all physical forms of administration and presentation, oral antiseptics on all its physical forms of administration and presentation, gels, pastes, mouthwashes, varnishes or even a combination of two or more compositions, associated to functional self-activated carboxylated metal phthalocyanines (octa or tetra). Such compositions for oral health, through synergistic action of functional self-activated carboxylated metal phthalocyanines (octa or tetra) promote formation and continuous release of reactive oxygen in suitable amounts for the described purposes, and may be used both directly by the patient or by a professional, in order to increase the efficiency of the product when compared to commercial products.

### DESCRIPTION OF THE DRAWINGS

This invention patent application will be described in detail, based on the figures listed below, in which:

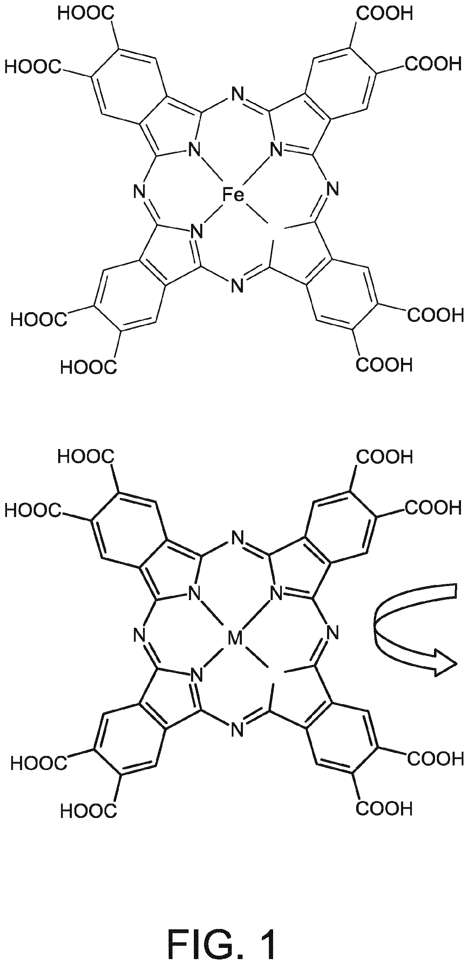

Figure 1 shows the chemical structure of the active agent, object of this patent, in which the reactive O₂ promotes pain reduction, accelerates coagulation and tissue healing, features antimicrobial action, inhibits the formation and removes the biofilm, inhibits formation and removes tartar and, lastly, promotes bleaching, returning the teeth to its natural color;

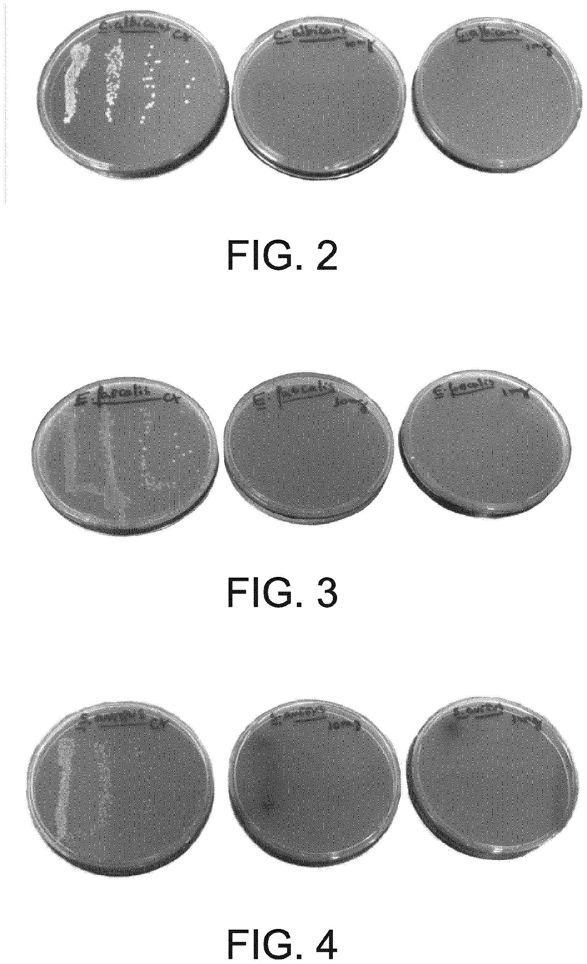

Figure 2 shows the application of Phthalocyanine on the *Candida albicans* (ATCC 10231) microorganism for 24 hours, using three containers where the first showed positive control, the second provided 10,000 mg Phthalocyanine and the third container showed 1,000 mg Phthalocyanine;
Figure 3 shows the application of Phthalocyanine on the *Enterococcus Faecalis* (ATCC 10231) microorganism for 24 hours, using three containers where the first showed positive control, the second provided 10,000 mg Phthalocyanine and the third container showed 1,000 mg Phthalocyanine;
Figure 4 shows the application of Phthalocyanine on the *Staphylococcus aureus* (ATCC 65381) microorganism for 24 hours, using three containers where the first showed positive control, the second provided 10,000 mg Phthalocyanine and the third container showed 1,000 mg Phthalocyanine;

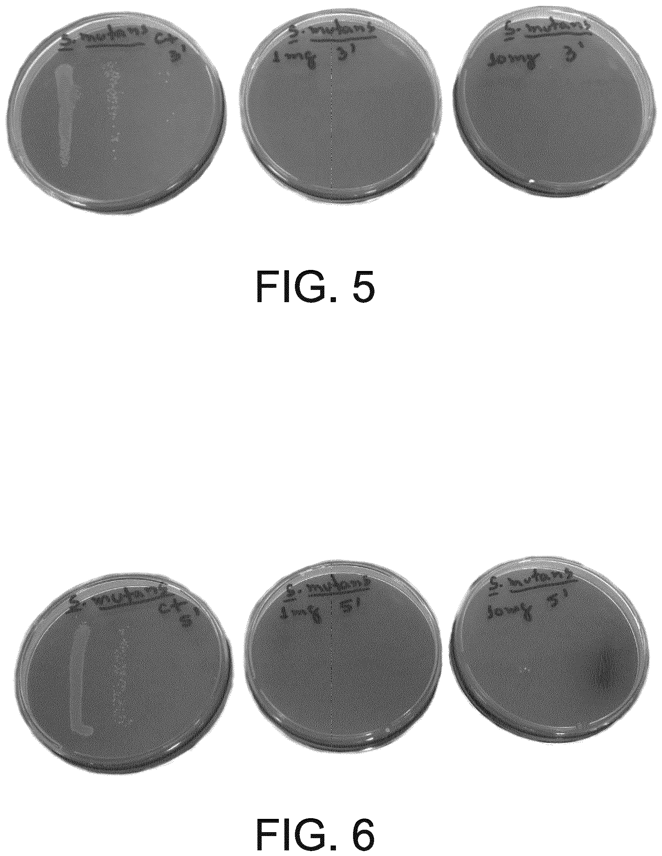

Figure 5 shows the application of Phthalocyanine on the *Streptococcus mutans* (ATCC 25175) microorganism for 3 minutes, using three containers where the first showed positive control, the second provided 10,000 mg Phthalocyanine and the third container showed 1,000 mg Phthalocyanine; and
Figure 6 shows the application of Phthalocyanine on the *Streptococcus mutans* (ATCC 25175) microorganism for 5 minutes, using three containers where the first showed positive control, the second provided 10,000 mg Phthalocyanine and the third container showed 1,000 mg Phthalocyanine.

### DETAILED DESCRIPTION OF THE INVENTION

This invention refers to a gel composition with the following ingredients: thickeners, moisteners, such as sorbitol, glycerin, polyethylene glycol and/or mixtures thereof, binders such as carboxymethyl cellulose, hydroxyethyl cellulose, xanthan gum, silica, thickener and/or mixtures thereof, conservatives (for example, methylparaben, propylparaben or mixtures thereof), acidifiers (typically phosphoric acid, citric acid, tartaric acid, maleic acid), essence (mint, etc.), flavoring agents (sodium saccharine or others).

The active ingredient comprises functional self-activated carboxy-metal-phthalocyanines (octa or tetra).

Concentrations of inactive ingredients are compliant with the market products and will be analyzed on each type of mixture in particular. The active ingredient - functional self-activated carboxy-metal-phthalocyanines (octa or tetra) features concentrations varying from 0,001% to 20,000%.

Preparation of gel composition: STEP (A): mix and homogenize separately low density carboxymethyl cellulose (CMC), glycerin and/or propylene glycol; STEP (B): solubilize separately sodium saccharine at 20,000% water and add to mixture A; STEP (C): solubilize separately sodium fluoride at 20% water and add to mixture A; STEP (D): solubilize separately methylparaben and propylparaben at 20,000% water and add to mixture A; STEP (E): solubilize separately functional self-activated carboxy-metal-phthalocyanines (octa or tetra) without glycerin and add to mixture A; STEP (F): thoroughly and slowly homogenize the mixture, preferably at vacuum conditions, until all material clusters are removed from the full mixture. (pH 6-8).

| GEL compound | Amounts % |
|---|---|
| Moistener (glycerin, Sorbitol, PEG 600) | 30,000 |
| Thickener (Carboxymethylcellulose, silica | 10,000 |
| Medium (Deionized Water) | 57,000 |
| Active Agent | 0,800 |
| Sweetener (Sodium Saccharin) | 0,100 |
| Preservative (Sodium Benzoate) | 1,000 |
| Flavoring Agent | 1,000 |
| pH corrector | 0,100 |

This invention refers to a varnish composition with the following ingredients: a base or matrix (natural resins such as colophony or synthetic resins, such as ethyl cellulose or hydroxypropyl methylcellulose), solvent (ethanol, other), fluorinated agents (sodium monofluoride phosphate, titanium tetrafluoride, stannous fluoride, calcium fluoride, amine fluoride, preferably sodium fluoride) and functional self-activated carboxy-metal-phthalocyanines (octa or tetra). Matrix concentrations, as per composition of this invention, features concentrations that may vary between 5,000% and 15,000%. Solvents comprise concentrations that vary between 70,000 to 90,000% and essence between 0,500% and 1,500%. The fluorinated agents added to this invention feature concentrations that may vary from 0 (zero) to 11.250 ppm.

The active ingredient, functional self-activated carboxy-metal-phthalocyanines (octa or tetra), features concentrations varying from 0,001% to 20,000%.

Preparation of varnish composition: STEP (A): mix and homogenize separately the matrix (artificial resin) and functional self-activated carboxy-metal-phthalocyanines (octa or tetra) to the solvent (ethanol); STEP (B): add to the mixture of STEP A sodium fluoride, and homogenize; STEP (C): thoroughly and slowly homogenize the mixture, preferably at vacuum conditions, until all material clusters are removed from the full mixture. Thoroughly and slowly homogenize the mixture, preferably at vacuum conditions, until all material clusters are removed from the full mixture.

| Varnish Composition | Amounts % |
|---|---|
| Matrix | 12,000 |
| Solvent | 82,000 |
| Sodium Fluoride | 2,500 |
| Active Agent | 1,500 |
| Flavoring Agent | 2,000 |

This invention refers to a composition of Oral Antiseptic that comprises inactive ingredients such as moisturizers such as sorbitol, glycerin, polyethylene glycol and/or mixtures thereof, conservatives (methylparaben, sodium benzoate, propylparaben, or any mixture thereof), acidifiers (phosphoric acid, citric acid, tartaric acid, maleic acid), essences (mint, etc.), flavoring agents (sodium saccharine or others), dyes. Active ingredients are comprised of fluorinated agents (sodium monofluoride phosphate, titanium tetrafluoride, stannous fluoride, amine fluoride, preferably sodium fluoride) and functional self-activated carboxy-metal-phthalocyanines (octa or tetra). The concentrations of moisturizing agents may vary from 2,000 to 15,000%. Conservatives used in the composition of this invention may vary between 0,010 and 0,500%. Other inactive ingredients shall have its concentration defined according to market needs.

Active ingredients such as fluorinated agents feature concentrations that may vary between 0,000 and 450,000 ppm. Functional self-activated carboxy-metal-phthalocyanines (octa or tetra) comprise concentrations that may vary between 0,001 to 20,000% of total composition. Other active ingredients such as antimicrobials shall have their concentration defined according to market needs and requirements.

Preparation of oral antiseptic composition: STEP (A): solubilize separately sodium fluoride at 20,000% water; STEP (B): solubilize separately sodium saccharine at 20,000% water and add to mixture A; STEP (C): solubilize separately methylparaben and propylparaben at 20.000% water and add to mixture A; STEP (D): solubilize separately functional self-activated carboxy-metal-phthalocyanines (octa or tetra) in glycerin and add to mixture A; STEP (E): mix and homogenize separately glycerin and/or propylene glycol to the aroma and add to mixture A; STEP (F): thoroughly and slowly homogenize the mixture, preferably at vacuum conditions. (pH 6-8).

| Antiseptic Composition | Amounts % |
|---|---|
| Moistener (glycerin, Sorbitol, PEG 600) | 5,000 |
| Active Agent | 0,100 |
| Medium (Deionized Water) | 94,000 |
| Sodium Fluoride | 0,050 |
| Sweetener (Sodium Saccharin) | 0,100 |
| Preservative (Sodium Benzoate) | 0,100 |
| Flavoring Agent | 0,500 |

This invention refers to a toothpaste composition with the following ingredients: Carboxymethyl cellulose, Glycerin, Deionized Water, Sodium fluoride, Sodium Saccharin, Sodium Benzoate, Sorbitol, Polyethylene Glycol - PEG 600, Silica, Sodium Lauryl Sulfate, Flavoring Agents.

The active ingredient comprises functional self-activated carboxy-metal-phthalocyanines (octa or tetra). Concentrations of inactive ingredients are compliant with the market products and will be analyzed on each type of mixture in particular.

The active ingredient, functional self-activated carboxy-metal-phthalocyanines (octa or tetra), features concentrations varying from 0,001% to 20,000%.

The process starts with the dissolution preparation of the Active Ingredient - PHTHALO in a moisturizer (Lauryl, Polyethylene Glycol) and then addition to the thickener, forming a pre-mixture of active ingredient/moisturizer/thickener.

This step occurs under slow stirring, between 45.000 to 200.000 rpm at 25°C, as to prevent formation of clusters, considering that the stirring remains until full dissolution of the ingredients, which lasts around 15 minutes.

Subsequently, the following components are added in a reactor to be homogenized, for 10 minutes, in a speed and temperature indicated by the equipment manufacturer: Deionized Water, Sodium fluoride, Sodium saccharin and Sodium Benzoate.

Then, Sorbitol and the Pre-mixture with the previously prepared active ingredient are added to the mixture, to be fully homogenized using a turbine, scraper and fan for 10 minutes, at a speed and temperature indicated by the equipment manufacturer (preferably in vacuum conditions).

Then, silica is added to the mixture, to be fully homogenized using a scraper and fan for 1 hour and 30 minutes, at a speed and temperature indicated by the equipment manufacturer, and then Triclosan (antiseptic) and Flavoring Agents are added to the mixture, for full homogenization using a scraper for 15 minutes, at a speed and temperature indicated by the equipment manufacturer.

After obtaining the toothpaste composition, occurred after homogenization of all components, as previously described, the pH correction takes place at a value between 6 and 8; through addition and homogenization of the pH corrector, at a speed and temperature indicated by the equipment manufacturer.

Subsequently the composition is filled into tubes.

| Formula components | Amounts % |
|---|---|
| Moisturizer (glycerin, Sorbitol, PEG 600, sodium lauryl sulfate) | 50,000 to 60,000 |
| Thickener (Carboxymethyl cellulose, silica) | 8,000 to 12,000 |
| Medium (Deionized Water) | 2,000 to 30,000 |
| Active ingredient - Anticaries agent (Sodium fluoride) | 0,250 to 0,500 |
| Sweetener (Sodium Saccharin, xylitol) | 0,500 to 1,500 |
| Preservative (Sodium Benzoate) | 0,100 to 0,500 |
| Abrasive (silica) | 5,000 to 15,000 |
| Surfactant (Sodium Lauryl sulfate) | 5,000 to 10,000 |
| Antiseptic (Triclosan, Cetylpyridinium chloride) | 0,100 to 0,300 |
| Flavoring Agent | 1,000 to 3,000 |
| pH corrector (Ortho-phosphoric acid, citric acid, sodium phosphate) | 0,100 to 2,000 |
| Active Agent | 0,100 to 1,000 |

Although the invention is detailed, it is important to understand that its application is not limited to details and steps described herein. The invention is capable of other modalities and to be practiced or performed in a variety of ways. It should be understood that the terminology used herein has descriptive purposes and is not limiting.

## Claims

1. Oral health composition, **characterized in that** it is in the form of a gel composition with the following ingredients: thickeners, moisturizers such as sorbitol, glycerin, polyethylene glycol and/or mixtures thereof, binders such as carboxymethyl cellulose, hydroxyethyl cellulose, xanthan gum, silica, thickener and/or mixtures thereof, conservatives (for example, methylparaben, propylparaben or mixtures thereof), acidifiers (typically phosphoric acid, citric acid, tartaric acid, maleic acid), essences (mint, etc.), flavoring agent (sodium saccharin, or others), said gel composition comprising 30,000% Moisturizer (glycerin, Sorbitol, PEG 600), 10,000% Thickener (Carboxymethyl cellulose, silica), 57,000% Medium (Deionized Water), 0,800% active agent, 0,100% Sweetener (Sodium saccharin), 1,000% Conservative (Sodium Benzoate), 1,000% Flavoring Agent and 0,100% pH Corrector.

2. Oral health composition, according to claim 1, wherein the active ingredient comprises functional self-activated carboxy-metal-phthalocyanines (octa or tetra).

3. Oral health composition, according to claims 1 and 2, wherein the active ingredient, functional self-activated carboxy-metal-phthalocyanines (octa/tetra), features a concentration varying between 0,001% and 20,000%.

4. Preparation process for oral health composition, of a gel-type composition, **characterized in that** it provides the following steps: (A) mix and homogenize separately low density carboxymethyl cellulose (CMC), glycerin and/or propylene glycol; (B) solubilize separately sodium saccharine at 20% water and add to mixture A; (C) solubilize separately sodium fluoride at 20% water and add to mixture A; (D) solubilize separately methylparaben and propylparaben at 20% water and add to mixture A; (E) solubilize separately functional self-activated carboxy-metal-phthalocyanines (octa or tetra) in glycerin and add to mixture A; (F) thoroughly and slowly homogenize the mixture at vacuum conditions until all material clusters are removed from the full mixture. (pH 6-8).

5. Oral health composition, **characterized in that** it is in the form of a varnish composition with the following ingredients: a base or matrix (natural resins such as colophony or synthetic resins, such as ethyl cellulose or hydroxypropyl methylcellulose), solvent (ethanol, other), fluorinated agents (sodium monofluoride phosphate, titanium tetrafluoride, stannous fluoride, calcium fluoride, amine fluoride, sodium fluoride) and functional self-activated carboxy-metal-phthalocyanines (octa or tetra), said composition featuring 12,000% Matrix, 82,000% Solvent, 2,500% Sodium Fluoride, 1,500% Active Agent and 2,000% Flavoring Agent.

6. Oral health composition, according to claim 5, wherein the matrix concentrations feature concentrations varying between 5,000 and 15,000%.

7. Oral health composition, according to claims 5 and 6, wherein the solvents have concentrations ranging from 70,000% to 90,000%, essence from 0,500% to 1,550% and fluorinated agents added from 0 (zero) to 11.250 ppm.

8. Oral health composition according to claims 5, 6 and 7, wherein the active ingredient, the functional self-activated carboxy-metal-phthalocyanines (octa/tetra), features concentrations ranging from 0,001 to 20,000%, and said antiseptic composition comprising 5,000% Moisturizer (glycerin, Sorbitol, PEG 600), 0,100% Active Agent, 94,000% Medium (Deionized Water), 0,050% Sodium Fluoride, 0,100% Sweetener (Sodium saccharin), 0,100% Conservative (Sodium Benzoate) and 0,500% Flavoring Agent.

9. Preparation process for oral health composition, of a varnish composition, wherein it provides (A) mix and homogenize separately the matrix (artificial resin) and functional self-activated carboxy-metal-phthalocyanines (octa or tetra) to the solvent (ethanol), add to the mixture A sodium fluoride, and thoroughly and slowly homogenize the mixture at vacuum conditions until all material clusters are removed from the full mixture.

10. Oral health composition, **characterized by** the fact that it is in the form of an oral antiseptic composition with inactive ingredients such as moisturizers such as sorbitol, glycerin, polyethylene glycol and/or mixtures thereof, conservatives (methylparaben, sodium benzoate, propylparaben or mixture thereof), acidifiers (phosphoric acid, citric acid, tartaric acid, maleic acid), essences (mint, etc.), flavoring agents (sodium saccharin or others) and dyes, the active ingredients comprising fluorinated agents (sodium monofluoride phosphate, titanium tetrafluoride, stannous fluoride, amine fluoride, sodium fluoride) and functional self-activated carboxy-metal-phthalocyanines (octa or tetra).

11. Oral health composition, according to claim 10, wherein the concentrations of moisturizing agents vary between 2,000 and 15,000%, and the conservatives used in the composition vary between 0,010 and 0,500%.

12. Oral health composition, according to claims 10 and 11, wherein the active ingredients such as fluorinated agents feature concentrations varying between 0,000 and 450,000 ppm, functional self-activated carboxy-metal-phthalocyanines (octa/tetra) feature a concentration varying between 0,001 and 20,000% of the total composition.

13. Preparation process for oral health composition, of an oral antiseptic composition, wherein it comprises the steps of (A) solubilize separately sodium fluoride at 20,000% water; (B) solubilize separately sodium saccharin at 20,000% water and add to mixture A; (C) solubilize separately methylparaben and propylparaben at 20,000% water and add to mixture A; (D) solubilize separately functional self-activated carboxy-metal-phthalocyanines (octa or tetra) in glycerin and add to mixture A; (E) mix and homogenize separately glycerin and/or propylene glycol to the aroma and add to mixture A; thoroughly and slowly homogenize the mixture at vacuum conditions (pH 6-8), the formula featuring 50,000% to 60,000% Moisturizer (Glycerin, Sorbitol, PEG 600), 8,000% to 12,000% Thickener (Carboxymethyl Cellulose, Silica), 2,000% to 4,900% Medium (Deionized Water), 0,250% to 0,500% Active Ingredient - Anticaries agent (Sodium Fluoride), 0,500% to 1,500% Sweetener (Sodium Saccharin, Xylitol), 0,100% to 0,500% Conservative (Sodium Benzoate), 0,300% to 1,000% Active Ingredient - Anti-Tartar Agent (TSPP), 5,000% to 15,000% Abrasive (Silica), 5,000% to 10,000% Surfactant (Sodium Lauryl Sulphate), 0,100% to 0,300% Antiseptic (Triclosan), 1,000% to 3,000% Flavoring Agent, 0,100% to 0,200% Pigment (Mica), 0,800% to 2,000% pH Corrector (Ortho-Phosphoric Acid) and 0,100% to 1,000% Active Agent.

14. Oral health composition, of a toothpaste composition, **characterized by** the fact that it comprises the following ingredients: Carboxymethyl Cellulose, Glycerin, Deionized Water, Sodium Fluoride, Sodium Saccharin, Sodium Benzoate, Sorbitol, Polyethylene Glycol - PEG 600, Silica, Sodium Lauryl Sulfate, Flavoring Agents.

15. Oral health composition, according to claim 14, wherein it provides a composition of functional self-activated carboxy-metal-phthalocyanines (octa/tetra).

16. Oral health composition, according to claims 14 and 15, wherein the active ingredient, the functional self-activated carboxy-metal-phthalocyanines (octa or tetra), features a concentration varying between 0,000001% and 20%.

17. Oral health composition, according to claims 1, 2, 3, 5, 6, 7, 8, 10, 11, 12, 14, 15 and 16, wherein it features an action based on continuous and localized formation of reactive oxygen, with no need for external activation by light, chemicals or electricity, except for oxygen, by functional self-activated carboxy-metal-phthalocyanines and active agent.

18. Oral health composition, according to claims 1, 2, 3, 5, 6, 7, 8, 10, 11, 12, 14, 15, 16 and 17, wherein said composition, through a synergistic action of oxygen from the air with functional self-activated carboxy-metal-phthalocyanines (octa or tetra), with metals Fe or Co are both administered in the encapsulated form.

19. Oral health composition, according to claims 1, 2, 3, 5, 6, 7, 8, 10, 11, 12, 14, 15, 16, 17 and 18, wherein said composition, through a synergistic action of oxygen from the air with functional self-activated carboxy-metal-phthalocyanines (octa or tetra), with metals Fe or Co are both administered in the molecular form.

20. Oral health composition, according to claims 1, 2, 3, 5, 6, 7, 8, 10, 11, 12, 14, 15, 16, 17, 18 and 19, wherein the active agent is associated to the fluorine and calcium complexes and remineralizing phosphate.

21. Oral health composition, according to claims 1, 2, 3, 5, 6, 7, 8, 10, 11, 12, 14, 15, 16, 17, 18 e 19 and 20, wherein the active agent is associated to the bleaching agent.

22. Oral health composition, according to claims 1, 2, 3, 5,6,7,8,10,11,12,14,15,16,17,18,19,20 and 21, wherein the active agent is associated to the desensitizing agents.
